(19) Europäisches Patentamt
European Patent Office
Office européen des brevets

(11) EP 1 944 065 A1

(12) **DEMANDE DE BREVET EUROPEEN**

(43) Date de publication:
**16.07.2008 Bulletin 2008/29**

(21) Numéro de dépôt: **07122764.9**

(22) Date de dépôt: **10.12.2007**

(51) Int Cl.:
***A61Q 19/08*** *(2006.01)* ***A61K 8/81*** *(2006.01)*
***A61K 8/87*** *(2006.01)* ***A61K 8/25*** *(2006.01)*
***A61K 8/89*** *(2006.01)*

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK RS**

(30) Priorité: **09.01.2007 FR 0752577**

(71) Demandeur: **L'Oréal**
**75008 Paris (FR)**

(72) Inventeur: **Cassin, Guillaume**
**91140, Villebon sur Yvette (FR)**

(74) Mandataire: **Kromer, Christophe et al**
**L'Oréal, DIPI, River Plaza**
**25-29 Quai Aulagnier**
**92665 Asnieres-sur-Seine (FR)**

(54) **Composition cosmétique contenant un polymère tenseur et des particules hybrides**

(57) L'invention concerne une composition cosmétique comprenant, dans un milieu physiologiquement acceptable, un polymère tenseur et des particules hybrides organique-minérale.

L'invention concerne également un procédé cosmétique de soin de la peau, plus particulièrement de la peau du visage, en particulier d'une peau ridée, comprenant l'application sur la peau de la composition.

EP 1 944 065 A1

## Description

La présente invention concerne une composition cosmétique, notamment une composition antirides, comprenant, dans un milieu physiologiquement acceptable, un polymère tenseur et des particules hybrides organique-minérale. Elle concerne également un procédé cosmétique de soin d'une peau ridée, destiné à atténuer les rides, comprenant l'application sur ladite peau d'une composition telle que définie ci-dessus.

Le domaine général de l'invention est donc celui du vieillissement de la peau.

Au cours du processus de vieillissement, une altération de la structure et des fonctions cutanées apparaît. Les principaux signes cliniques observés sont l'apparition de rides et de ridules liées à un relâchement cutané. L'homme de l'art sait qu'un tel relâchement peut être corrigé de façon immédiate par l'application d'un agent tenseur sur la peau.

A ce jour, l'utilisation de nombreux polymères tenseurs pour traiter les rides est connue de l'homme de l'art. On pense en particulier aux polymères siliconés greffés acryliques ou les réseaux interpénétrés de polymères pour lisser les rides par effet tenseur , notamment décrits dans les documents EP1038519, FR2843025.

Malheureusement, les compositions contenant de tels agents tenseurs, bien que présentant un effet tenseur immédiat très satisfaisant ont l'inconvénient majeur de présenter un effet limité dans le temps.

Par conséquent, il existe un besoin pour des compositions cosmétiques présentant à la fois une excellente efficacité et pour lesquelles l'effet tenseur soit durable. Or, la Demanderesse a découvert que l'inclusion de particules hybrides organique-minérale dans des compositions comprenant un polymère tenseur permettait d'améliorer les propriétés mécaniques du film tenseur polymérique pour lui permettre notamment de suivre les mimiques sans se fissurer, et ainsi d'améliorer la rémanence de l'effet tenseur sur la peau.

La présente invention a donc pour objet une composition cosmétique comprenant, dans un milieu physiologiquement acceptable, un polymère tenseur et des particules hybrides organique-minérale.

L'invention a aussi pour objet un procédé cosmétique de soin de la peau, plus particulièrement de la peau du visage, en particulier de la peau ridée, comprenant l'application topique sur la peau d'une composition telle que définie précédemment.

La composition et le procédé selon l'invention sont en particulier destiné à lisser la peau humaine du visage et/ou du corps et/ou à diminuer ou effacer les signes du vieillissement cutané, en particulier à réduire ou effacer les rides et/ou les ridules de la peau.

Les constituants de la composition selon l'invention seront maintenant décrits plus en détail.

### Polymère tenseur

Par polymère tenseur, on entend, selon l'invention, un polymère susceptible d'avoir un effet tenseur, c'est à dire pouvant tendre la peau et par cet effet de tension lisser la peau et faire diminuer voire disparaître de façon immédiate les rides et les ridules.

Plus particulièrement, cette expression désigne tout polymère soluble ou dispersible dans l'eau à une température allant de 25°C à 50°C à la concentration de 7% en poids dans l'eau ou à la concentration maximale à laquelle il forme un milieu d'apparence homogène et produisant à cette concentration de 7% ou à cette concentration maximale dans l'eau une rétractation de plus de 15 % dans le test décrit ci-après.

La concentration maximale à laquelle il forme un milieu d'apparence homogène est déterminée à ± 10% près et de préférence à ± 5% près.

On entend par 'milieu d'apparence homogène' un milieu ne présentant pas d'agrégats visibles à l'oeil nu.

Pour la détermination de ladite concentration maximale, le polymère tenseur est ajouté progressivement dans l'eau sous agitation à la défloculeuse à une température allant de 25°C à 50°C, puis le mélange est maintenu sous agitation pendant une heure. On observe ensuite après 24 heures si le mélange ainsi préparé est d'apparence homogène (absence d'agrégats visibles à l'oeil nu).

L'effet tenseur peut être caractérisé par un test *in vitro* de rétractation.

On prépare préalablement et tel que décrit précédemment un mélange homogène de le polymère tenseur dans l'eau, à la concentration de 7% en poids ou à la concentration maximale définie précédemment.

30μl du mélange homogène est déposé sur une éprouvette rectangulaire (10x40mm , donc présentant une largeur initiale $L_0$ de 10 mm) d'élastomère ayant un module de 20 MPa et une épaisseur de 100μm.

Après 3h de séchage à 22±3°C et 40±10% d'humidité relative HR, l'éprouvette d'élastomère présente une largeur rétractée, notée $L_{3h}$ due à la tension exercée par le polymère tenseur déposé.

L'effet tenseur (ET) dudit polymère est alors quantifié de la façon suivante :

$$\text{'ET'} = (L_0 - L_{3h} / L_0)\text{x}100 \text{ en } \%$$

avec $L_o$ = largeur initiale 10mm
et $L_{3h}$ = largeur après 3h de séchage

La polymère tenseur peut notamment être choisi parmi les polymères synthétiques, les protéines végétales et leurs hydrolysats, et leurs mélanges.
Ces différentes catégories de polymères tenseurs seront maintenant décrites.

a) Les polymères synthétiques

« Polymérique synthétique » caractérise tout polymère obtenu chimiquement ou par production dans un organisme des éléments nécessaires à cette production.
Le polymère tenseur synthétique peut être, dans la composition selon l'invention, soit solubilisé ou dispersé dans un liquide polaire ou apolaire. Il peut en particulier être solubilisé dans l'eau ou bien être sous forme de dispersion aqueuse de particules.
Les polymères tenseurs peuvent notamment se trouver sous la forme de réseaux de polymères interpénétrés (IPNs), de polycondensats, des polymères acryliques, des polymères siliconés greffés ou de polymères en étoiles.

Réseau de polymères interpénétrés

Selon une première variante, la composition selon la présente invention comprend au moins un polymère tenseur synthétique de type réseau de polymères interpénétrés.
Par "réseau de polymères interpénétrés" au sens de la présente invention, on entend un mélange de deux polymères enchevêtrés, obtenu par polymérisation et/ou réticulation simultanée de deux types de monomères, le mélange obtenu ayant une température de transition vitreuse unique.
Des exemples d'IPNs convenant à une mise en oeuvre dans la présente invention, ainsi que leur procédé de préparation, sont par exemple décrits dans les brevets US-6,139,322 et US-6,465,001.
De préférence, l'IPN selon l'invention comprend au moins un polymère polyacrylique et, plus préférentiellement, il comprend en outre au moins un polyuréthane ou un copolymère de fluorure de vinylidène et d'hexafluoropropylène. Selon une forme d'exécution préférée, l'IPN selon l'invention comprend un polymère polyuréthane et un polymère polyacrylique. De tels IPNs sont notamment ceux de la série Hybridur qui sont disponibles dans le commerce auprès de la société AIR PRODUCTS.
Un IPN particulièrement préféré se trouve sous la forme d'une dispersion aqueuse de particules ayant une taille moyenne, en poids, comprise entre 90 et 110 nm et une taille moyenne, en nombre, d'environ 80 nm. Cet IPN a de préférence une température de transition vitreuse, Tg, qui va d'environ -60°C à +100°C. Un IPN de ce type est notamment commercialisé par la société AIR PRODUCTS sous la dénomination commerciale Hybridur X-01602. Un autre IPN convenant à une utilisation dans la présente invention est référencé Hybridur X18693-21.
D'autres IPNs convenant à une mise en oeuvre dans la présente invention comprennent les IPNs constitués du mélange d'un polyuréthane avec un copolymère de fluorure de vinylidène et d'hexafluoropropylène. Ces IPNs peuvent notamment être préparés comme décrit dans le brevet US-5,349,003. En variante, ils sont disponibles dans le commerce sous forme de dispersion colloïdale dans l'eau, dans un rapport du copolymère fluoré au polymère acrylique comprise entre 70:30 et 75:25, sous les dénominations commerciales KYNAR RC-10,147 et KYNAR RC-10,151 auprès de la société ATOFINA.

Polycondensat

La composition peut selon une deuxième variante comprendre comme polymère tenseur synthétique au moins un polycondensat.
Des polymères sous forme de polycondensats ayant un effet antiride ont notamment été décrits dans la demande WO 98/29092.
Comme polycondensats, on peut citer les polyuréthannes, notamment anioniques, cationiques, non ioniques ou amphotères, les polyuréthannes-acryliques, les polyuréthannes-polyvinylpyrrolidones, les polyester-polyuréthannes, les polyéther-polyuréthannes, les polyurées, et leurs mélanges.
Le polyuréthanne peut être, par exemple, un copolymère polyuréthanne, ou polyurée/uréthanne ou polyurée, aliphatique, cycloaliphatique ou aromatique, comportant, seul ou en mélange,

- au moins une séquence d'origine polyester aliphatique linéaire ou ramifié et/ou cycloaliphatique et/ou aromatique, et/ou
- au moins une séquence d'origine polyéther aliphatique et/ou cycloaliphatique et/ou aromatique, et/ou

- au moins une séquence comportant des groupes fluorés.

Les polyuréthannes peuvent être également obtenus à partir de polyesters, ramifiés ou non, ou d'alkydes comportant des hydrogènes mobiles que l'on modifie par réaction avec un diisocyanate et un composé organique bifonctionnel (par exemple dihydro, diamino ou hydroxyamino), comportant en plus soit un groupement acide carboxylique ou carboxylate, soit un groupement acide sulfonique ou sulfonate, soit encore un groupement amine tertiaire neutralisable ou un groupement ammonium quaternaire. On peut également citer les polyesters, les polyesters amides, les polyesters à chaîne grasse, les polyamides, et les résines époxyesters.
En vue de former un polyuréthanne, on peut citer comme monomère porteur de groupement anionique pouvant être utilisé lors de la polycondensation, l'acide diméthylolpropionique, l'acide trimellitique ou un dérivé tel que l'anhydride trimellitique, le sel de sodium de l'acide sulfo-3 pentanediol, le sel de sodium de l'acide 5-sulfo 1,3-benzènedicarboxylique.
Parmi les polycondensats, on peut citer les polymères commercialisés sous les dénominations commerciales AVALURE UR405 et AVALURE UR450 par la société NOVEON.

Polymères acryliques

Le polymère tenseur selon l'invention peut être choisi parmi les copolymères linéaires statistiques acryliques de poids moléculaire inférieur à 600000 g/mol, de préférence un poids moléculaire en poids comprise entre 15 000 et 600 000 g/mol et contenant au moins 70% d'un monomère ayant une température de transition vitreuse Tg supérieure à 40°C (de préférence > 60°C) dont l'homopolymère correspondant est insoluble dans l'eau à 25°C et au moins un monomère acide (méth)acrylique. Ce copolymère peut également contenir un monomère non majoritaire de Tg inférieure à 40°C.
Ces copolymères présentent généralement une température globale de transition vitreuse supérieure ou égale à 45 °C.
On utilise de préférence les copolymères constitués :

• de 70 à 90% en poids d'au moins un monomère choisi parmi le styrène, l'acrylate de benzyle, l'acrylate de cyclohexyle, l'acrylate de tertiobutyle, l'acrylate d'isobornyle l'acrylate de norbornyle, le méthacrylate de méthyle, le méthacrylate d'éthyle, le méthacrylate d'isobutyle, le méthacrylate de cyclohexyle, le méthacrylate de benzyle, le méthacrylate de tertiobutyle, le méthacrylate d'isobornyle et le méthacrylate de norbornyle ;

• de 10 à 30% en poids d'acide (méth)acrylique.

Préférentiellement, on utilise les copolymères constitués :

• de 70 à 90% en poids d'au moins un monomère choisi parmi le styrène, méthacrylate de méthyle et méthacrylate de cyclohexyle ;

• de 10 à 30% en poids d'acide (méth)acrylique.

Parmi les polymères cités ci-dessus, on préférera particulièrement:

- Les copolymères de méthacrylate de méthyle / acide méthacrylique ; les copolymères de méthacrylate de méthyle / acide acrylique, lesdits copolymères contenant entre 70 et 90 % en poids de méthacrylate de méthyle ;
- Les copolymères de méthacrylate d'éthyle/ acide méthacrylique ; les copolymères de méthacrylate d'éthyle / acide acrylique, lesdits copolymères contenant entre 70 et 90 % en poids de méthacrylate d'éthyle ;
- Les copolymères de méthacrylate d'isobutyle/ acide méthacrylique ; les copolymères de méthacrylate d'isobutyle / acide acrylique, lesdits copolymères contenant entre 70 et 90 % en poids de méthacrylate d'isobutyle ;
- Les copolymères de méthacrylate de benzyle / acide méthacrylique ; les copolymères de méthacrylate de benzyle / acide acrylique, lesdits copolymères contenant entre 70 et 90 % en poids de méthacrylate de benzyle ;
- Les copolymères d'acrylate de benzyle / acide méthacrylique ; les copolymères d'acrylate de benzyle / acide acrylique, lesdits copolymères contenant entre 70 et 90 % en poids d'acrylate de benzyle ;
- Les copolymères de méthacrylate de cyclohexyle / acide méthacrylique ; les copolymères de méthacrylate de cyclohexyle / acide acrylique, lesdits copolymères contenant entre 70 et 90 % en poids de méthacrylate de cyclohexyle ;
- Les copolymères d'acrylate de cyclohexyle / acide méthacrylique ; les copolymères d'acrylate de cyclohexyle / acide acrylique, lesdits copolymères contenant entre 70 et 90 % en poids d'acrylate de cyclohexyle ;
- Les copolymères de méthacrylate de tertio-butyle/ acide méthacrylique ; les copolymères de méthacrylate de tertio-butyle / acide acrylique, lesdits copolymères contenant entre 70 et 90 % en poids de méthacrylate de tertio-butyle ;

- Les copolymères d'acrylate de tertio-butyle/ acide méthacrylique ; les copolymères d'acrylate de tertio-butyle / acide acrylique, lesdits copolymères contenant entre 70 et 90 % en poids d'acrylate de tertio-butyle ;
- Les copolymères de méthacrylate d'isobornyle/ acide méthacrylique ; les copolymères de méthacrylate d'isobornyle / acide acrylique, lesdits copolymères contenant entre 70 et 90 % en poids de méthacrylate d'isobornyle ;
- Les copolymères d'acrylate d'isobornyle / acide méthacrylique; les copolymères d'acrylate d'isobornyle / acide acrylique, lesdits copolymères contenant entre 70 et 90 % en poids d'acrylate d'isobornyle ;
- Les copolymères de méthacrylate de norbornyle/ acide méthacrylique ; les copolymères de méthacrylate de norbornyle / acide acrylique, lesdits copolymères contenant entre 70 et 90 % en poids de méthacrylate de norbornyle ;
- Les copolymères d'acrylate de norbornyle / acide méthacrylique ; les copolymères d'acrylate de norbornyle / acide acrylique, lesdits copolymères contenant entre 70 et 90 % en poids d'acrylate de norbornyle et ;
- Les copolymères de styrène / acide méthacrylique ; les copolymères de styrène / acide acrylique, lesdits copolymères contenant entre 70 et 90 % en poids de styrène.

On peut également utiliser les polymères acryliques vendus sous les dénominations « NEOCRYL XK 90 » et « NEOCRYL XK 99 » par la société AVECIA NEORESINS.

<u>Polymère siliconé greffé</u>

Parmi les agents tenseurs polymériques synthétiques utilisés dans la composition selon l'invention, on peut en variante citer les polymères siliconés greffés notamment, tels que définis dans la demande EP-1038519. Il peut s'agir plus particulièrement d'un polymère comprenant une chaîne principale de silicone ou polysiloxane (polymère de Si-O-) sur laquelle se trouve greffé, à l'intérieur de ladite chaîne ainsi qu'éventuellement à l'une au moins de ses extrémités, au moins un groupement organique ne comportant pas de silicone.

Les polymères à squelette polysiloxanique greffé par des monomères organiques non-siliconés selon l'invention peuvent être des produits commerciaux existants, ou encore être obtenus selon tout moyen connu de l'homme de l'art, en particulier par réaction entre (i) une silicone de départ correctement fonctionnalisée sur un ou plusieurs de ses atomes de silicium et (ii) un composé organique non-siliconé lui-même correctement fonctionnalisé par une fonction qui est capable de venir réagir avec le ou les groupements fonctionnels portés par ladite silicone en formant une liaison covalente ; un exemple classique d'une telle réaction est la réaction d'hydrosylilation entre des groupements $\equiv$Si-H et des groupements vinyliques $CH_2$=CH-, ou encore la réaction entre des groupements thio-fonctionnels -SH et ces mêmes groupements vinyliques.

Des exemples de polymères à squelette polysiloxanique greffé par des monomères organiques non-siliconés convenant à une mise en oeuvre de la présente invention, ainsi que leur mode particulier de préparation, sont notamment décrits dans les demandes de brevets EP-A-0582152, WO 93/23009 et WO 95/03776 dont les enseignements sont totalement inclus dans la présente description à titre de références non limitatives.

Selon un mode particulièrement préféré de réalisation de la présente invention, le polymère siliconé, à squelette polysiloxanique greffé par des monomères organiques non-siliconés, mis en oeuvre comprend le résultat de la copolymérisation radicalaire entre d'une part au moins un monomère organique anionique non-siliconé présentant une insaturation éthylénique et/ou un monomère organique hydrophobe non-siliconé présentant une insaturation éthylénique et d'autre part une silicone présentant dans sa chaîne au moins un groupement fonctionnel capable de venir réagir sur lesdites insaturations éthyléniques desdits monomères non-siliconés en formant une liaison covalente, en particulier des groupements thio-fonctionnels.

Selon la présente invention, lesdits monomères anioniques à insaturation éthylénique sont de préférence choisis, seuls ou en mélanges, parmi les acides carboxyliques insaturés, linéaires ou ramifiés, éventuellement partiellement ou totalement neutralisés sous la forme d'un sel, ce ou ces acides carboxyliques insaturés pouvant être plus particulièrement l'acide acrylique, l'acide méthacrylique, l'acide maléique, l'anhydride maléique, l'acide itaconique, l'acide fumarique et l'acide crotonique. Les sels convenables sont notamment les sels d'alcalins, d'alcalino-terreux et d'ammonium. On notera que, de même, dans le polymère siliconé greffé final, le groupement organique à caractère anionique qui comprend le résultat de l'(homo)polymérisation radicalaire d'au moins un monomère anionique de type acide carboxylique insaturé peut être, après réaction, post-neutralisé avec une base (soude, ammoniaque,...) pour l'amener sous la forme d'un sel.

Selon la présente invention, les monomères hydrophobes à insaturation éthylénique sont de préférence choisis, seuls ou en mélanges, parmi les esters d'acide acrylique d'alcanols et/ou les esters d'acide méthacrylique d'alcanols. Les alcanols sont de préférence en $C_1$-$C_{18}$ et plus particulièrement en $C_1$-$C_{12}$. Les monomères préférentiels sont choisis dans le groupe constitué par le (méth)acrylate d'isooctyle, le (méth)acrylate d'isononyle, le 2-éthylhexyl(méth)acrylate, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth)acrylate de stéaryle ou leurs mélanges.

Une famille de polymères siliconés à squelette polysiloxanique greffé par des monomères organiques non-siliconés convenant particulièrement bien à la mise en oeuvre de la présente invention est constituée par les polymères siliconés comportant dans leur structure le motif de formule (I) suivant :

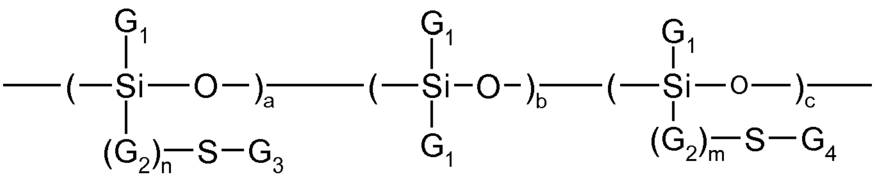

(I)

dans lequel les radicaux $G_1$, identiques ou différents, représentent l'hydrogène ou un radical alkyle en $C_1$-$C_{10}$ ou encore un radical phényle ; les radicaux $G_2$ , identiques ou différents, représentent un groupe alkylène en $C_1$-$C_{10}$ ; $G_3$ représente un reste polymérique résultant de l'(homo)polymérisation d'au moins un monomère anionique à insaturation éthylénique ; $G_4$ représente un reste polymérique résultant de l'(homo)polymérisation d'au moins un monomère hydrophobe à insaturation éthylénique ; m et n sont, indépendamment l'un de l'autre, égaux à 0 ou 1 ; a est un nombre entier allant de 0 et 50 ; b est un nombre entier pouvant être compris entre 10 et 350, c est un nombre entier allant de 0 à 50 ; sous réserve que l'un des paramètres a et c soit différent de 0.

De préférence, le motif de formule (I) ci-dessus présente au moins l'une, et encore plus préférentiellement l'ensemble, des caractéristiques suivantes:

- les radicaux $G_1$ désignent un radical alkyle en $C_1C_{10}$ ;
- n est non nul, et les radicaux $G_2$ représentent un radical divalent en $C_1$-$C_3$ ;
- $G_3$ représente un radical polymérique résultant de 1'(homo)polymérisation d'au moins un monomère du type acide carboxylique à insaturation éthylénique, de préférence l'acide acrylique et/ou l'acide méthacrylique ;
- $G_4$ représente un radical polymérique résultant de l'(homo)polymérisation d'au moins un monomère du type (méth) acrylate d'alkyle en $C_1$-$C_{10}$.

Des exemples de polymères siliconés greffés répondant à la formule (I) sont ainsi notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et/ou du type poly(méth)acrylate d'alkyle.

Ces polymères sont référencés sous la dénomination CTFA "polysilicone-8".

Il peut ainsi s'agir d'un polydiméthyl siloxane greffé propylthio(polyacrylate de méthyle/méthacrylate de méthyle/acide méthacrylique) ou d'un polydiméthyl siloxane greffé propylthio(polyacrylate de méthyle), propylthio(polyméthacrylate de méthyle) et propylthio(polyacide méthacrylique). En variante, il peut s'agir d'un polydiméthyl siloxane greffé propylthio(polyméthacrylate d'isobutyle) et propylthio(polyacide méthacrylique). On utilise de préférence un polydiméthyl siloxane greffé propylthio(polyacrylate de méthyle/méthacrylate de méthyle/acide méthacrylique).

De tels polymères siliconés greffés sont notamment vendus par la Société 3M sous les dénominations commerciales VS 80, VS 70 ou LO21.

De préférence, la masse moléculaire en nombre des polymères siliconés à squelette polysiloxanique greffé par des monomères organiques non-siliconés de l'invention varie de 10 000 à 1 000 000 environ, et encore plus préférentiellement de 10 000 à 100 000 environ.

<u>Polymère en étoile</u>

Selon une autre possibilité encore, l'agent tenseur polymérique synthétique pouvant être utilisé dans la composition selon l'invention peut comprendre au moins un polymère de structure en "étoile" représenté par la formule (11) suivante :

$$A\text{-}[(M_1)_{p1} - (M_2)_{p2} .... (Mi)_{pj}]_n \qquad (II)$$

dans laquelle :

- A représente un centre multifonctionnel, de fonctionnalité "n", n étant un entier supérieur à 2, en particulier supérieur à 5.
- $[(M_1)_{p1} - (M_2)_{p2} .... (M)_{pj}]$ représente une chaîne polymérique, aussi appelée "branche", constituée de monomères

$M_i$ polymérisés, identiques ou différents, ayant un indice de polymérisation pj, chaque branche étant identique ou différente, et étant greffée de manière covalente sur ledit centre A,

- i est supérieur ou égal à 1, et pj est supérieur ou égal à 2;

ledit polymère comprenant un ou plusieurs monomères $M_i$ dont l'homopolymère correspondant présente une Tg supérieure ou égale à environ 10 °C, de préférence supérieure ou égale à 15 °C, et encore mieux supérieure ou égale à 20 °C; et ce ou ces monomères $M_i$ étant présents en une quantité minimale d'environ 45 % en poids, de préférence en une quantité variant entre 55 et 99 % en poids, et encore mieux entre 75 et 90 % en poids, par rapport au poids total de l'ensemble des monomères du polymère final. Ces polymères, ainsi que leur procédé de préparation, sont notamment décrits dans le document EP 1 043 345.

b) Les protéines végétales et leurs hydrolysats

Des exemples de protéines végétales et hydrolysats de protéines végétales utilisables comme agents tenseurs selon l'invention sont constitués des protéines et hydrolysats de protéines de maïs, de seigle, de froment, de sarrasin, de sésame, d'épeautre, de pois, de fève, de lentille, de soja et de lupin.
Avantageusement, le polymère tenseur est choisi parmi les polymères synthétiques de type réseau de polymère interpénétrés, notamment les réseaux interpénétrés de polyuréthane et de polyacrylique, et les polymères siliconés à squelette polysiloxanique greffé par des monomères organiques non-siliconés, tels que décrits précédemment.
Le polymère tenseur peut être compris dans la composition selon l'invention en une teneur allant de 0,01 à 20% en poids de matière active, de préférence de 1% à 10% en poids de matière active, par rapport au poids total de la composition. Par « matière active », on entend le polymère sans le milieu dans lequel il se trouve éventuellement solubilisé ou en dispersion.

Les particules hybrides organique-minérale

On entend par « particules hybrides organique - minérale », des particules composites constituées d'au moins un polymère synthétique constituant une matrice de ces particules, et d'au moins deux particules colloïdales minérales incluses dans la matrice de ces particules.
Par particules colloïdales minérales, on entend, des particules de charges minérales de diamètre moyen en nombre allant de 0,1 à 100 nm, et de préférence de 3 à 30 nm.
Les particules colloïdales minérales peuvent être choisies parmi les particules colloïdales de silice, d'oxyde de cérium, d'oxyde de zirconium, d'alumine, de carbonate de calcium, de sulfate de baryum, de sulfate de calcium, d'oxyde de zinc et de dioxyde de titane, les particules colloïdales de platine, les particules colloïdales mixtes comme par exemple les dioxydes de titane enrobés de silice, ou les particules colloïdales composites de silice-alumine. On utilisera de préférence des particules colloïdales de silice ou des particules colloïdales composites silice-alumine.
Dans un mode de réalisation alternatif de l'invention, les particules colloïdales minérales peuvent être choisies parmi les particules colloïdales de silicate mixte. On utilisera alors de préférence des phyllosilicates, à savoir des silicates ayant une structure dans laquelle les tétraèdres SiO4 sont organisés en feuillets entre lesquels se trouvent enfermés les cations métalliques. Une famille de silicates particulièrement préférée est celle des laponites. Les laponites sont des silicates de magnésium, de lithium et de sodium ayant une structure en couches semblable à celle des montmorillonites. La laponite est la forme synthétique du minéral naturel appelé "hectorite".
Les particules colloïdales minérales constituent de 5 à 80 % en poids du poids total des particules hybrides organique-minérales, de préférence de 20 à 80 % en poids, et préférentiellement de 30 à 70 % en poids.
Ces particules colloïdales minérales peuvent être présentes dans les particules hybrides selon l'invention sous la forme d'inclusions totales, partielles ou en surface.
Les polymères constituant la matrice des particules hybrides organique-minérale peuvent être choisis parmi les polyuréthannes ; les polymères radicalaires dont les polymères acryliques, et de préférence parmi les polymères acryliques.
Les polymères radicalaires peuvent notamment comprendre un ou plusieurs monomères choisis parmi les acides (méth)acrylique, l'éthylène, le styrène, le méthyl styrène, les esters d'alcool vinylique et de monoacide carboxylique en $C_1$-$C_{18}$ comme l'acétate de vinyle, le propionate de vinyle, le n-butyrate de vinyle, le laurate de vinyle, le stéarate de vinyle ; l'acide maléique, l'acide fumarique, l'acide itaconique, les (méth)acrylates d'alkyle en $C_1$-$C_8$ (de préférence en $C_1$-$C_4$) comme le (méth)acrylate de méthyle, le (méth)acrylate d'éthyle, le (méth)acrylate de n-butyle, le (méth)acrylate d'isobutyle, le (méth)acrylate de 2-éthylhexyle. De préférence, les polymères radicalaires comprennent un ou plusieurs monomères choisis parmi les (méth)acrylates d'alkyle en $C_1$-$C_8$ (notamment en $C_1$-$C_4$).
Les particules hybrides organique-minérale peuvent comprendre de 20 % à 95 % en poids, par rapport au poids desdites particules, de polymère synthétique (formant la matrice desdites particules), de préférence de 20 % à

80 % en poids, et préférentiellement de 30 % à 70 % en poids. Le complément à 100 % en poids est constitué des particules colloïdales minérales.

De préférence, les particules hybrides organique-minérale sont choisies parmi les particules hybrides polymère acrylique-silice.

Avantageusement, les particules hybrides organique-minérale ont un diamètre moyen en volume allant de 0,03 à 1 $\mu$m, et préférentiellement de 0,05 à 0,5 $\mu$m. Elles peuvent se présenter sous la forme d'une solution ou en suspension dans un liquide polaire ou apolaire, ou bien sous forme sèche redispersable dans un solvant cosmétique.

Avantageusement, les particules hybrides organique-minérale se présentent sous forme de dispersion aqueuse.

Les particules hybrides organique - minérale peuvent être présentes dans la composition en une quantité de matière active allant de 0,01 à 30 % en poids total de la composition, de préférence de 1 % à 20 %.

De telles particules hybrides organique-minérale sont notamment décrites dans les demandes WO 2006/048167, WO 2006/072464 et US 2004/0171728. Elle sont notamment formées par polymérisation en émulsion des monomères du polymère formant la matrice en présence des particules colloïdales minérales.

Comme exemple de particules hybrides organique-minérale, on peut citer les particules composites silice-copolymère acrylique en dispersion aqueuse , comme celles commercialisées sous les noms ACNANO DS 1000 X et NANOCOMPOSITE R2/768 par la société BASF.

La composition selon l'invention est généralement adaptée à une application topique sur la peau et comprend donc généralement un milieu physiologiquement acceptable, c'est-à-dire un milieu compatible avec la peau et/ou ses phanères. Il s'agit de préférence d'un milieu cosmétiquement acceptable, c'est-à-dire qui présente une couleur, une odeur et un toucher agréables et qui ne génère pas d'inconforts inacceptables (picotements, tiraillements, rougeurs), susceptibles de détourner la consommatrice d'utiliser cette composition.

La composition selon l'invention peut se présenter sous toutes les formes galéniques classiquement utilisées pour une application topique et notamment sous forme de dispersions du type lotion ou gel aqueux, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou de dispersions cire/phase aqueuse. Ces compositions sont préparées selon les méthodes usuelles.

Selon un mode préféré de réalisation de l'invention, la composition se présente sous forme d'une émulsion H/E ou d'un gel aqueux.

Cette composition peut en outre contenir divers adjuvants couramment utilisés dans le domaine cosmétique, tels que des émulsionnants comme les esters d'acides gras et de polyéthylène glycol, les esters d'acide gras et de sorbitane éventuellement polyoxyéthylénés, les alcools gras polyoxyéthylénés et les esters ou éthers d'acide gras et de sucres tel que le sucrose ou le glucose ; des charges ; des conservateurs ; des séquestrants ; des parfums ; et des épaississants et/ou des gélifiants, en particulier les polyacrylamides, les homo- et copolymères acryliques et les homo- et copolymères d'acide acrylamido méthylpropane sulfonique.

Bien entendu, l'homme du métier veillera à choisir ce ou ces éventuels composés additionnels et/ou leur quantité de manière telle que les propriétés anti-rides de la composition selon l'invention ne soient pas, ou substantiellement pas, altérées par l'adjonction envisagée.

La composition selon l'invention peut également contenir des actifs anti-âge à effet complémentaire à l'association selon l'invention, tels qu'au moins un composé choisi parmi les agents desquamants, les agents hydratants, les agents stimulant la prolifération et/ou la différenciation des kératinocytes, les agents stimulant la synthèse du collagène et/ou de l'élastine ou prévenant leur dégradation, les agents dépigmentants, les agents anti-glycation, les agents stimulant la synthèse de glycosaminoglycannes, les agents dermo-décontractants ou myorelaxants, les agents anti-oxydants et anti-radicalaires, et leurs mélanges.

Des exemples de tels actifs sont : le rétinol et ses dérivés tels que le palmitate de rétinyle ; l'acide ascorbique et ses dérivés tels que l'ascorbyl phosphate de magnésium et le glucoside d'ascorbyle ; le tocophérol et ses dérivés tels que l'acétate de tocophéryle ; l'acide nicotinique et ses précurseurs tels que la nicotinamide ; l'ubiquinone ; le glutathion et ses précurseurs tels que l'acide L-2-oxothiazolidine-4-carboxylique ; les extraits de plantes et notamment les extraits de criste marine et de feuille d'olivier ; les extraits d'algues et en particulier de laminaires ; les extraits bactériens ; les sapogénines telles que la diosgénine et les extraits de Dioscorées, en particulier de Wild Yam, en contenant ; les $\alpha$-hydroxyacides ; les $\beta$-hydroxyacides, tels que l'acide salicylique et l'acide n-octanoyl-5-salicylique ; les oligopeptides et pseudodipeptides et leurs dérivés acylés, en particulier l'acide {2-[acetyl-(3-trifluoromethyl-phenyl)-amino]-3-methyl-butyrylamino} acétique et les lipopeptides commercialisés par la société SEDERMA sous les dénominations commerciales Matrixyl 500 et Matrixyl 3000 ; le lycopène ; et leurs mélanges.

L'application de la composition selon l'invention se fait selon les techniques habituelles, par exemple par application (notamment de crèmes, de gels, de sérums, de lotions) sur la peau destinée à être traitée, en particulier la

peau du visage et/ou du cou, notamment la peau du contour de l'oeil. Dans le cadre de ce procédé, la composition peut être, par exemple, une composition de soin ou une composition de maquillage, en particulier de fond de teint.

L'invention va maintenant être décrite en référence aux exemples suivants donnés à titre illustratif et non limitatif.

## EXEMPLES

**Exemples 1 et 2 : Compositions cosmétiques : sérums antirides**

On a préparé 2 sérums selon l'invention (exemples 1A et 2A) et 2 sérums ne faisant pas partie de l'invention (exemples 1B et 2B), ces derniers ne contenant pas de particules hybride organique-minérale.

**Exemple 1A (invention) :** Sérum antirides contenant 3,5% d'Hybridur et 3,5% de particules hybrides organique-minérale.

**[0082]** - *Phase A*

| | |
|---|---|
| Eau | 76.90g |
| Copolymère méthyl vinyl éther/anhydride maléique réticulé par 1,9-décadiène (Antaron ST 06 de chez ISP) | 0.20 g |
| Gomme de Xanthane (Keltrol T de chez Kelco) | 0.20 g |
| Conservateur | 0.55 g |

- *Phase B*

| | |
|---|---|
| Eau | 1.00 g |
| Triéthanolamine | 0.20 g |

- *Phase C*

| | |
|---|---|
| Copolymère acrylamide/acrylamido 2-méthyl propane sulfonate de sodium en émulsion inverse à 40% dans isoparaffine/eau (Sepigel 305 de chez Seppic) | 1.00 g |
| Conservateur | 0.30 g |

- *Phase D*

| | |
|---|---|
| Dispersion aqueuse à 40 % en poids de particules d'un réseau interpénétré de polymères polyuréthane et polyacrylique (Hybridur® 875 de chez Air Products) | 8.63 g |
| Dispersion aqueuse de particule hybride polymère acrylique-silice (AcNano DS 1000 X de chez BASF) | 10.00 g |

**Exemple 1B (hors invention) :** Sérum antirides contenant 7% d'Hybridur 875.

**[0083]** - *Phase A*

| | |
|---|---|
| Eau | 78.25 g |
| Copolymère méthyl vinyl éther/anhydride maléique réticulé par 1,9-décadiène (Antaron® ST 06 de chez ISP) | 0.20 g |
| Gomme de Xanthane (Keltrol® T de chez Kelco) | 0.20 g |
| Conservateur | 0.55 g |

- *Phase B*

| | |
|---|---|
| Eau | 1.00 g |
| Triéthanolamine | 0.20 g |

- *Phase C*

| | |
|---|---|
| Copolymère acrylamide/acrylamido 2-méthyl propane sulfonate de sodium en émulsion inverse à 40% dans isoparaffine/eau (Sepigel® 305 de chez Seppic) | 1.00 g |
| Conservateur | 0.30 g |

- *Phase D*

| | |
|---|---|
| Dispersion aqueuse à 40 % en poids de particules d'un réseau interpénétré de polymères polyuréthane et polyacrylique (Hybridur® 875 de chez Air Products) | 7.30 g |

Le sérum antirides a été préparé de la manière suivante :
On chauffe à 75°C environ, sous agitation, la phase A, puis on verse la phase B dans la phase A. Ensuite, on arrête le chauffage tout en maintenant l'agitation jusqu'au retour à température ambiante et l'on ajoute alors les phases C et D. Une légère agitation est ensuite maintenue pendant 30 minutes.

**Exemple 2A (invention) :** Sérum antirides contenant 3,5% d'Avalure et 3,5% de particules hybrides organique-minérale.

**[0085]** - *Phase A*

| | |
|---|---|
| Eau | 75.55 g |
| Copolymère méthyl vinyl éther/anhydride maléique réticulé par 1,9-décadiène (Antaron® ST 06 de chez ISP) | 0.20 g |
| Gomme de Xanthane (Keltrol® T - Kelco) | 0.20 g |
| Conservateur | 0.55 g |

- *Phase B*

| | |
|---|---|
| Eau | 1.00 g |
| Triéthanolamine | 0.20 g |

- *Phase C*

| | |
|---|---|
| Copolymère acrylamide/acrylamido 2-méthyl propane sulfonate de sodium en émulsion inverse à 40% dans isoparaffine/eau (Sepigel® 305 de chez Seppic) | 1.00 g |
| Conservateur | 0.30 g |

- *Phase D*

| | |
|---|---|
| Dispersion aqueuse à 35 % de polyuréthane (Avalure ®UR405 de chez NOVEON) | 10.00 g |
| Dispersion aqueuse de particules hybrides polymère acrylique-silice (AcNano DS 1000 X - BASF) | 10.00 g |

**Exemple 2B (hors invention) :** Sérum antirides contenant 3.5 % Avalure UR405.

**[0086]** - *Phase A*

| | |
|---|---|
| Eau | 85.55 g |
| Copolymère méthyl vinyl éther/anhydride maléique réticulé par 1,9-décadiène (Antaron ®ST 06 de chez ISP) | 0.20 g |
| Gomme de Xanthane (Keltrol® T de chez Kelco) | 0.20 g |
| Conservateur | 0.55 g |

- *Phase B*

| | |
|---|---|
| Eau | 1.00 g |
| Triéthanolamine | 0.20 g |

- *Phase C*

| | |
|---|---|
| Copolymère acrylamide/acrylamido 2-méthyl propane sulfonate de sodium en émulsion inverse à 40% dans isoparaffine/eau (Sepigel ®305 de chez Seppic) | 1.00 g |
| Conservateur | 0.30 g |

- *Phase D*

| | |
|---|---|
| Dispersion aqueuse à 35 % de polyuréthane (Avalure® UR405 de chez NOVEON) | 10.00 g |

**Exemple 3 : Mise en évidence de l'amélioration des propriétés de rémanence des formules selon l'invention**

L'essai consiste à solliciter en compression jusqu'à rupture un matériau (en l'occurrence les crèmes anti-rides des exemples 1A et 1B) déposé en surface d'une mousse souple et déformable. L'utilisation de ce support en mousse permet d'imposer une importante déformation au matériau déposé en surface, et donc une quantification de sa résistance à la rupture. La sollicitation mécanique en compression est exercée à l'aide d'un poinçon cylindrique de diamètre 1 mm ; la vitesse de déplacement du poinçon étant de 0,1 mm/s. L'essai est réalisé à l'aide d'un analyseur de texture TA-XT2i commercialisé par la société Stable Micro System. Il est ainsi obtenu une courbe force F (en N) en fonction du déplacement d (en mm) à partir de laquelle il est possible de déterminer le point de rupture du matériau. La figure 1 en annexe présente un tel exemple de courbe de force en fonction du déplacement.

Le paramètre Wrupt (l'énergie à rupture en $J/m^2$) est retenu pour quantifier la résistance à la rupture du matériau. Le paramètre correspondant à la surface sous la courbe F=f(d) / surface du poinçon.

Le substrat est constitué d'une mousse néoprène de 13 mm d'épaisseur. Le matériau (composition antirides) est déposé sur ce substrat de façon à obtenir après un séchage de 24 h un film d'une épaisseur de 15 à 30 μm. Les dépôts ont été réalisés à l'aide d'un tireur de film déposant 650 μm humide.

Les résultats obtenus sont les suivants :

| Matériau | Wrupt ($J/m^2$) |
|---|---|
| Exemple 1A<br>Sérum contenant 3,5% d'Hybridur 875 et 3,5% de particules hybride organique-minérale (AcNano DS 1000 X - BASF) | 417 ± 38 |
| Exemple 1B (comparatif)<br>Sérum contenant 7% d'Hybridur 875 | 59 ± 7 |
| Exemple 2A<br>Sérum contenant 3,5% d'Avalure UR405 et 3,5% de particules hybride organique - minérale (AcNano DS 1000 X - BASF) | 1978 ± 520 |
| Exemple 2B (comparatif)<br>Sérum contenant 3,5% d'Avalure UR405 | 395 ± 30 |

On constate que les particules hybrides utilisées dans les compositions selon l'invention des exemples 1A et 2A ont un rôle renforçateur, ce rôle de renfort s'illustrant par une augmentation de l'énergie à la rupture. Il en résulte un effet tenseur plus rémanent des compositions des exemples 1A et 2A en comparaison de l'effet tenseur des compositions 2B et 2C ne faisant pas partie de l'invention. En effet, si l'effet tenseur est lié à la formation d'un dépôt rigide, on comprend que la pérennité de cet effet est liée à la résistance mécanique de ce dépôt.

Il a par ailleurs été vérifié sur un panel de 6 femmes que les compositions des exemples 1A et 2A présentent un effet tenseur satisfaisant.

**Exemple 4** : Sérum antirides contenant 3,5% de polymère silicone greffé et 3,5% de particules hybrides organique-minérale.

On prépare la composition suivante :

- *Phase A*

| | |
|---|---|
| Eau | qsp 100 g |
| Copolymère méthyl vinyl éther/anhydride maléique réticulé par 1,9-décadiène (Antaron ST 06 de chez ISP) | 0.20 g |
| Gomme de Xanthane (Keltrol T de chez Kelco) | 0.20 g |
| Conservateur | 0.55 g |

- *Phase B*

| | |
|---|---|
| Eau | 1.00 g |
| Triéthanolamine | 0.20 g |

- *Phase C*

| | |
|---|---|
| Copolymère acrylamide/acrylamido 2-méthyl propane sulfonate de sodium en émulsion inverse à 40% dans isoparaffine/eau (Sepigel 305 de chez Seppic) | 1.00 g |
| Conservateur | 0.30 g |

- *Phase D*

| | |
|---|---|
| Poly dimethyl / methyl siloxane a groupements propyl thio-3 acrylate de methyle / methacrylatede methyle / acide methacrylique (LO21 DRY de chez 3M) | 8.63 g |
| Dispersion aqueuse de particule hybride polymère acrylique-silice (AcNano DS 1000 X de chez BASF) | 10.00 g |

La composition appliquée sur la peau confère un effet anti-rides durable.

## Revendications

1. Composition cosmétique comprenant, dans un milieu physiologiquement acceptable, un polymère tenseur et des particules hybrides organique-minérale.

2. Composition selon la revendication 1, **caractérisée en ce que** le polymère tenseur est choisi parmi les polymères synthétiques, les protéines végétales et leurs hydrolysats, et leurs mélanges.

3. Composition selon l'une quelconque des revendications précédentes, **caractérisée en ce que** le polymère tenseur est compris dans la composition en une teneur allant de 1 à 10% en poids de matière active, par rapport au poids total de la composition.

4. Composition selon l'une quelconque des revendications précédentes, **caractérisée en ce que** le polymère tenseur est choisi parmi les polymères siliconés à squelette polysiloxanique greffé par des monomères organiques non-siliconés

5. Composition selon la revendication précédente, **caractérisée en ce que** le polymère siliconé comporte dans sa structure le motif de formule (I) suivant :

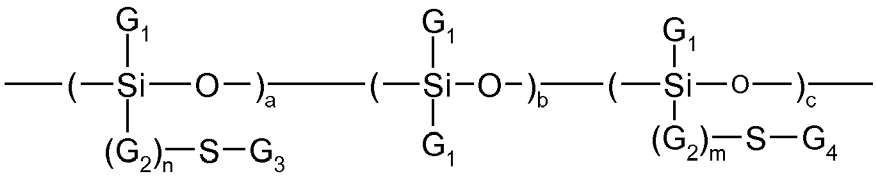

(I)

dans lequel les radicaux $G_1$ , identiques ou différents, représentent l'hydrogène ou un radical alkyle en $C_1$-$C_{10}$ ou encore un radical phényle ; les radicaux $G_2$ , identiques ou différents, représentent un groupe alkylène en $C_1$-$C_{10}$ ; $G_3$ représente un reste polymérique résultant de l'(homo)polymérisation d'au moins un monomère anionique à insaturation éthylénique ; $G_4$ représente un reste polymérique résultant de l'(homo)polymérisation d'au moins un monomère hydrophobe à insaturation éthylénique ; m et n sont, indépendamment l'un de l'autre, égaux à 0 ou 1 ; a est un nombre entier allant de 0 et 50 ; b est un nombre entier pouvant être compris entre 10 et 350, c est un nombre entier allant de 0 à 50 ; sous réserve que l'un des paramètres a et c soit différent de 0.

**6.** Composition selon la revendication précédente, **caractérisée en ce que** le motif de formule (I) présente au moins l'une, et encore plus préférentiellement l'ensemble, des caractéristiques suivantes :

- les radicaux $G_1$ désignent un radical alkyle en $C_1$-$C_{10}$ ;
- n est non nul, et les radicaux $G_2$ représentent un radical divalent en $C_1$-$C_3$;
- $G_3$ représente un radical polymérique résultant de l'(homo)polymérisation d'au moins un monomère du type acide carboxylique à insaturation éthylénique, de préférence l'acide acrylique et/ou l'acide méthacrylique ;
- $G_4$ représente un radical polymérique résultant de l'(homo)polymérisation d'au moins un monomère du type (méth)acrylate d'alkyle en $C_1$-$C_{10}$.

**7.** Composition selon l'une des revendications 5 ou 6, **caractérisée en ce que** les polymères siliconés greffés répondant à la formule (I) sont des polydiméthylsiloxanes (PDMS) sur lesquels sont greffés, par l'intermédiaire d'un chaînon de raccordement de type thiopropylène, des motifs polymères du type acide poly(méth)acrylique et/ou du type poly (méth)acrylate d'alkyle, notamment d'alkyle en $C_1$-$C_3$, voire en $C_1$.

**8.** Composition selon l'une des revendications 4 à 7, **caractérisée en ce que** le polymère siliconé greffé est un polydiméthyl siloxane greffé propylthio(polyacrylate de méthyle/méthacrylate de méthyle/acide méthacrylique).

**9.** Composition selon l'une des revendications 1 à 3, **caractérisée en ce que** le polymère tenseur est un polymère de type réseau de polymères interpénétrés.

**10.** Composition selon la revendication précédente, **caractérisée en ce que** le réseau de polymères interpénétrés comprend un polymère polyuréthane et un polymère acrylique.

**11.** Composition selon l'une quelconque des revendications précédentes, **caractérisée en ce que** les particules hybrides organique-minérale sont constituées d'une matrice de polymère synthétique dans laquelle sont inclues au moins des particules minérales colloïdales.

**12.** Composition selon la revendication précédente, **caractérisée en ce que** les particules minérales colloïdales ont un diamètre moyen en nombre allant de 0,1 à 100 nm, et de préférence de 3 à 30 nm.

**13.** Composition selon l'une des revendications 11 ou 12, **caractérisée en ce que** les particules colloïdales minérales sont choisies parmi les particules colloïdales de silice, d'oxyde de cérium, d'oxyde de zirconium, d'alumine, de carbonate de calcium, de sulfate de baryum, de sulfate de calcium, d'oxyde de zinc et de dioxyde de titane, les particules colloïdales de platine, les particules colloïdales mixtes comme par exemple les dioxydes de titane enrobés de silice, ou les particules colloïdales composites de silice-alumine, les particules colloïdales de silicate mixte.

**14.** Composition selon l'une quelconque des revendications 11 à 13, **caractérisée en ce que** les particules colloïdales minérales sont choisies parmi les particules colloïdales de silice ou les particules colloïdales composites silice-alumine.

**15.** Composition selon l'une quelconque des revendications 11 à 14, **caractérisée en ce que** les particules colloïdales

minérales constituent de 5 à 80 % en poids du poids total des particules hybrides organique-minérales, de préférence de 20 à 80 % en poids, et préférentiellement de 30 à 70 % en poids.

**16.** Composition selon l'une quelconque des revendications 11 à 15, **caractérisée en ce que** les polymères constituant la matrice des particules hybrides organique-minérale peuvent être choisis parmi les polyuréthannes ; les polymères radicalaires dont les polymères acryliques, et de préférence parmi les polymères acryliques.

**17.** Composition selon l'une quelconque des revendications 11 à 16, **caractérisée en ce que** le polymère constituant la matrice des particules hybrides organique-minérale comprend un ou plusieurs monomères choisis parmi les (méth)acrylates d'alkyle en $C_1$-$C_8$ , de préférence en $C_1$-$C_4$.

**18.** Composition selon l'une quelconque des revendications précédentes, **caractérisée en ce que** les particules hybrides organique-minérale sont choisies parmi les particules hybrides polymère acrylique-silice.

**19.** Composition selon l'une quelconque des revendications précédentes, **caractérisée en ce que** les particules hybrides organique-minérales ont un diamètre moyen en volume allant de 0,03 à 1 $\mu$m, et préférentiellement de 0,05 à 0,5 $\mu$m.

**20.** Composition selon l'une quelconque des revendications précédentes, **caractérisée en ce que** les particules hybrides organique-minérale se présentent sous forme de dispersion aqueuse.

**21.** Composition selon l'une quelconque des revendications précédentes, **caractérisée en ce que** les particules hybrides organique-minérale sont présentes en une quantité de matière active allant de 0,01 à 30 % en poids, par rapport au poids total de la composition, de préférence allant de 1 % à 20 % en poids.

**22.** Composition selon l'une quelconque des revendications précédentes, **caractérisée en ce qu'**elle se présente sous forme d'une émulsion H/E ou d'un gel aqueux.

**23.** Procédé cosmétique de soin de la peau, comprenant l'application sur ladite peau d'une composition selon l'une quelconque des revendications 1 à 22.

**24.** Procédé selon la revendication précédente, **caractérisé en ce que** la composition est appliquée sur une peau ridée.

**25.** Procédé selon la revendication 23 ou 24, **caractérisé en ce qu'**il est destiné à atténuer les rides.

Office européen des brevets

# RAPPORT DE RECHERCHE EUROPEENNE

Numéro de la demande
EP 07 12 2764

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | EP 1 588 694 A1 (L'OREAL) 26 octobre 2005 (2005-10-26)<br>* page 11, ligne 1 - page 13, ligne 4 *<br>----- | 1-25 | INV.<br>A61Q19/08<br>A61K8/81<br>A61K8/87<br>A61K8/25<br>A61K8/89 |
| A | US 2003/215476 A1 (L'OREAL) 20 novembre 2003 (2003-11-20)<br>* alinéa [0013] - alinéa [0025] *<br>* alinéa [0033] - alinéa [0035] *<br>* alinéa [0042] - alinéa [0044] *<br>----- | 1-25 | |
| D,A | EP 1 038 519 A1 (L'OREAL) 27 septembre 2000 (2000-09-27)<br>* le document en entier *<br>----- | 4-8 | |
| A | FR 2 843 025 A (L'OREAL) 6 février 2004 (2004-02-06)<br>* exemples *<br>----- | 9,10 | |
| A | US 5 977 215 A (AIR PRODUCTS) 2 novembre 1999 (1999-11-02)<br>* exemples *<br>----- | 9,10 | DOMAINES TECHNIQUES RECHERCHES (IPC) |
| A,D | WO 2006/072464 A (BASF) 13 juillet 2006 (2006-07-13)<br>* le document en entier *<br>----- | 1,11-20 | A61K |
| A,D | US 2004/171728 A1 (BASF) 2 septembre 2004 (2004-09-02)<br>* le document en entier *<br>----- | 1,11-20 | |

Le présent rapport a été établi pour toutes les revendications

| Lieu de la recherche | Date d'achèvement de la recherche | Examinateur |
|---|---|---|
| Munich | 14 février 2008 | Irwin, Lucy |

3

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
........................................................................
& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 07 12 2764

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

14-02-2008

| Document brevet cité au rapport de recherche | Date de publication | Membre(s) de la famille de brevet(s) | Date de publication |
|---|---|---|---|
| EP 1588694 A1 | 26-10-2005 | AT 362750 T | 15-06-2007 |
| | | DE 602005001170 T2 | 17-01-2008 |
| | | ES 2286770 T3 | 01-12-2007 |
| | | FR 2868949 A1 | 21-10-2005 |
| | | JP 2005306871 A | 04-11-2005 |
| US 2003215476 A1 | 20-11-2003 | AUCUN | |
| EP 1038519 A1 | 27-09-2000 | AT 328579 T | 15-06-2006 |
| | | BR 0000714 A | 02-05-2001 |
| | | DE 60028435 T2 | 06-06-2007 |
| | | ES 2265884 T3 | 01-03-2007 |
| | | FR 2791257 A1 | 29-09-2000 |
| | | JP 2000297027 A | 24-10-2000 |
| | | KR 20010006848 A | 26-01-2001 |
| FR 2843025 A | 06-02-2004 | AUCUN | |
| US 5977215 A | 02-11-1999 | AUCUN | |
| WO 2006072464 A | 13-07-2006 | AU 2005324052 A1 | 13-07-2006 |
| | | CA 2592311 A1 | 13-07-2006 |
| | | CN 101098896 A | 02-01-2008 |
| | | DE 102005000918 A1 | 20-07-2006 |
| | | EP 1838740 A1 | 03-10-2007 |
| | | KR 20070103423 A | 23-10-2007 |
| US 2004171728 A1 | 02-09-2004 | AT 375370 T | 15-10-2007 |
| | | BR 0210566 A | 03-08-2004 |
| | | CA 2449887 A1 | 03-01-2003 |
| | | CN 1518566 A | 04-08-2004 |
| | | DE 10129537 A1 | 09-01-2003 |
| | | DK 1401902 T3 | 14-01-2008 |
| | | WO 03000760 A1 | 03-01-2003 |
| | | EP 1401902 A1 | 31-03-2004 |
| | | HU 0401878 A2 | 28-12-2004 |
| | | JP 2004532347 T | 21-10-2004 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 1038519 A
- FR 2843025
- US 6139322 A
- US 6465001 B
- US 5349003 A
- WO 9829092 A
- EP 0582152 A
- WO 9323009 A
- WO 9503776 A
- EP 1043345 A
- WO 2006048167 A
- WO 2006072464 A
- US 20040171728 A